(19) Europäisches Patentamt
European Patent Office
Office européen des brevets

(11) Publication number : **0 558 321 A1**

(12) **EUROPEAN PATENT APPLICATION**

(21) Application number : **93301415.1**

(22) Date of filing : **25.02.93**

(51) Int. Cl.[5] : **A61K 31/445,** A61K 31/40, C07D 295/092, C07D 487/04, // (C07D487/04, 209:00)

(30) Priority : **27.02.92 JP 41473/92**

(43) Date of publication of application : **01.09.93 Bulletin 93/35**

(84) Designated Contracting States : **AT BE CH DE DK ES FR GB GR IE IT LI LU MC NL PT SE**

(71) Applicant : **SANWA KAGAKU KENKYUSHO CO., LTD.**
**No. 35, Higashi-sotobori-cho**
**Higashi-ku Nagoya-shi Aichi-ken (JP)**

(72) Inventor : **Kurono, Masayasu, c/o Sanwa Kagaku Kenkyusho Co., Ltd., Higashi-sotobori-cho 35 Higashi-ku, Nagoya-shi, Aichi-ken (JP)**
Inventor : **Baba, Yutaka, c/o Sanwa Kagaku Kenkyusho Co., Ltd., Higashi-sotobori-cho 35 Higashi-ku, Nagoya-shi, Aichi-ken (JP)**
Inventor : **Oka, Mitsuru, c/o Sanwa Kagaku Kenkyusho Co., Ltd., Higashi-sotobori-cho 35 Higashi-ku, Nagoya-shi, Aichi-ken (JP)**
Inventor : **Honda, Naoki, c/o Sanwa Kagaku Kenkyusho Co., Ltd., Higashi-sotobori-cho 35 Higashi-ku, Nagoya-shi, Aichi-ken (JP)**
Inventor : **Matsumoto, Yukiharu, c/o Sanwa Kagaku Kenkyusho Co., Ltd., Higashi-sotobori-cho 35 Higashi-ku, Nagoya-shi, Aichi-ken (JP)**
Inventor : **Kakigami, Takuji, c/o Sanwa Kagaku Kenkyusho Co., Ltd., Higashi-sotobori-cho 35 Higashi-ku, Nagoya-shi, Aichi-ken (JP)**
Inventor : **Iwata, Noriyuki, c/o Sanwa Kagaku Kenkyusho Co., Ltd., Higashi-sotobori-cho 35 Higashi-ku, Nagoya-shi, Aichi-ken (JP)**
Inventor : **Ishiwata, Yoshiro, c/o Sanwa Kagaku Kenkyusho Co., Ltd., Higashi-sotobori-cho 35 Higashi-ku, Nagoya-shi, Aichi-ken (JP)**
Inventor : **Ohtuka,Tamaki c/o Sanwa Kagaku Kenkyusho Co., Ltd., Higashi-sotobori-cho 35 Higashi-ku, Nagoya-shi, Aichi-ken (JP)**
Inventor : **Mitani, Takahiko, c/o Sanwa Kagaku Kenkyusho Co., Ltd., Higashi-sotobori-cho 35 Higashi-ku, Nagoya-shi, Aichi-ken (JP)**
Inventor : **Sawai, Kiichi, c/o Sanwa Kagaku Kenkyusho Co., Ltd., Higashi-sotobori-cho 35 Higashi-ku, Nagoya-shi, Aichi-ken (JP)**

(74) Representative : **Diamond, Bryan Clive et al**
**Gee & Co., Chancery House, Chancery Lane**
**London WC2A 1QU (GB)**

(54) **Antivirally active N-cycloalkyl alkanol compounds.**

(57) The compounds are of the general formula

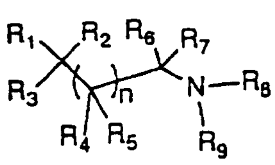

··· (I)

or

EP 0 558 321 A1

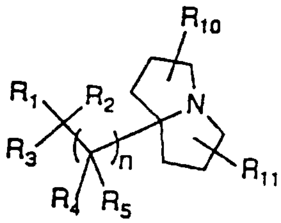

... (II)

where $R_1$ is -OH, amino or -SH, $R_2$ is H, α- or β-naphthyl or optionally substituted phenyl, $R_3$ is mono-, di- or tricycloalkyl of C6-16, optionally substituted, $R_{4-7}$ are H or alkyl, $R_{8-9}$ are alkyl or may form a 6-membered ring or a 5-membered ring with $R_6$ and $R_7$, $R_{10}$ and $R_{11}$ are alkyl, and $\underline{n}$ is 0 or an integer.

Synthesis examples are given, and for the preparation of starting materials.

Pharmaceutical compositions for oral, topical or injectable administration contain the compounds or their acceptable salts.

The compounds have excellent antiviral activity as illustrated against herpes virus and may also be effective against influenza, DNA, RNA and HIV viruses.

The present invention relates to an antivirally active compound used for preventing and treating viral infections ascribable to various DNA viruses, RNA viruses and retroviruses.

So far, many cholinolytic drugs for treating Parkinsonism have been developed, and pharmaceuticals having related structures have also been produced. However, few or no antiviral compounds having these compounds as their fundamental skeletons are known.

Compounds that are known to be efficacious against Parkinsonism and confirmed to be resistant to influenza virus are limited to Norakin, Palcopan, Akineton and Antiperkin (see "Acta. Virol.", 28, pp. 501-507 (1984)), and so are very small in number.

The working mechanism of how Norakin produces an antiviral action on influenza virus has been said to reside in the inhibition of hemagglutinin, which is the surface protein of influenza virus, but to lack in anti-viral activity against herpes virus ("J. Gen. Virol.", 67, pp. 1115-1122 (1986)).

In recent years, there has been enormous progress in the research and development of antiviral agents or drugs. In particular, antiviral agents based on nucleic acid - which are represented by acyclovir - have been developed and found to be clinically efficacious against herpes virus infections. However, another problem has arisen in connection with resistant virus (e.g., thymidine kinase negative ones) infections ("Oral. Surg. Oral. Med. Oral. Pathol.", Vol. 67, pp. 427-432 (1989)). In addition, these nucleic acid type of antiviral drugs have generally been known to be inefficacious against RNA viruses represented by influenza virus.

Among antiviral agents of types that are other than the nucleic acid type and efficacious against DNA viruses, for instance, phosphonoformate (PFA for short; see "Nippon Rinsho", Vol. 47(2), pp. 390-394 (1989)), phosphonoacetate (PAA for short; see "Nippon Rinsho", Vol. 47(2), pp. 390-394 (1989)) and cicloxolone ("Journal of Antimicrobial Chemotherapy", Vol. 18, Suppl. B. pp. 185-200 (1986)) have been known. However, problems with PFA and PAA are that they have side effects such as renal disorders and anemia, whereas cicloxolone has not an antiviral activity high-enough for therapy.

Among another group of antival agents that are efficacious against RNA viruses, for instance, Amantadine ("Shonika Shinryo", Vol. 54(4), pp. 988-994 (1991)), Remantadine ("Shonika Shinryo", Vol. 54(4), pp. 988-994 (1991)) and LY253963 (a thiadiazol derivative; "Shonika Shinryo", Vol. 54(4), pp. 988-994 (1991)) have been known. Amantadine and remantadine have been shown to be efficacious against influenza A virus but not against influenza B virus, and pose a grave problem - a side effect on the central nervous system. LY253963 has been shown to be resistant to influenza virus in animal tests, but its clinical efficacy has yet to be verified.

In recent years, patients with immunodeficiency diseases induced by organ transplantation, cancer chemotherapy and HIV infections have been on the increase, posing a grave medical problem. Viral infections in such patients are so diverse in type that they cannot often be treated with existing antiviral drugs. Thus, much is now expected of the development of much improved antiviral drugs.

It is therefore a first object of the invention to provide an antivirally active composition that is much more efficacious against DNA viruses, RNA viruses and retroviruses. A second object of the invention is to provide an antivirally active composition that contains as a main active ingredient a compound which is different in chemical structure from existing antiviral substances, thereby enabling it to be efficacious against viruses resistant to such existing antiviral substances.

Of medicaments for treating Parkinsonism, Norakin, Palcopan, Akineton and Antiperkin have been confirmed to have an anti influenza virus action ("Acta Virol.", 28, pp. 501-507 (1984)).

However, Norakin is a mixture of diastereoisomers, and we have now discovered that the anti influenza virus action of Norakin can be enhanced by separating the diastereoisomers from it.

On the other hand, 1-azabicyclo[3.3.0]octane derivatives, inclusive of 2-oxopyrrolidine compounds and their salts (JP-A-61-254587), organoplatinum complexes (JP-A-61-229893) and cephalosporin derivatives and their salts (JP-A-62-16487), have been widely used as partial structures of various pharmaceuticals.

We have therefore made further studies with a view to discovering compounds that have basically 1-azabicyclo[3.3.0] octane skeletons and novel structures that, until now, are not found in existing antivirally active compounds, and have consequently found that some novel compounds are of less cytotoxicity but have a much-more excellent anti-influenza virus activity.

The present invention provides antivirally active 3-piperidino-1-propanol derivatives, 1-(1-azabicyclo[3.3.0]octan -5-yl)methanol derivatives, 2-(1-azabicyclo[3.3.0]octan-5-yl)-1-ethanol derivatives and 2-(1-azabicyclo[3.3.0]octan-5-yl)-1-propanol derivatives which are structurally represented by the following general formulae I and II:

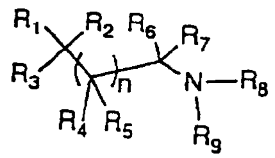

... (I)

wherein:

$R_1$ is selected from hydroxyl, amino and thiol groups,

$R_2$ is selected from a hydrogen atom, an α-naphthyl or β-naphthyl group and phenyl which may be substituted and represented by the formula:

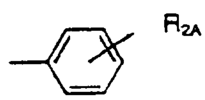

where $R_{2A}$ is selected from hydrogen, lower alkyl, halogeno, halogenoalkyl, $-OR_{2B}$, $-CO-R_{2C}$ and $-N-R_{2D}R_{2E}$, wherein $R_{2B}$ represents hydrogen or lower alkyl, and $R_{2C}$, $R_{2D}$ and $R_{2E}$ each represents lower alkyl,

$R_3$ is selected from monocycloalkyl, dicycloalkyl and tricycloalkyl groups which have each 6 to 16 carbon atoms and may have a substituent,

$R_{4-7}$ each each selected from hydrogen and lower alkyl,

$R_{8-9}$ are each lower alkyl, or may form a six-membered ring, or may form a five-membered ring with $R_6$ and $R_7$, and

n is 0 or a natural number,

and

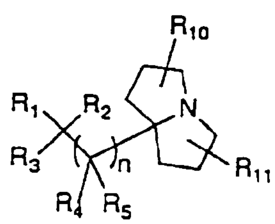

... (II)

wherein

$R_1$, $R_2$, $R_3$, $R_4$, $R_6$ and n have the same meanings as defined above, and

$R_{10}$ and $R_{11}$ each represent lower alkyl. These derivatives may be used in the form of their pharmaceutically acceptable salts.

The term "lower alkyl" means having up to 5, preferably up to 4, carbon atoms.

By way of example but not by way of limitation, the 3-piperidino-1-propanol derivatives represented by Formula I may include (1R*)-1-phenyl-3-piperidino-1-((3'S*)-3-tricyclo [2.2.1.0]heptyl)propan-1-ol, (1R*)-1-phenyl-3-piperidino-1-((3'R*)-3-tricyclo[2.2.1.0]heptyl)propan-1-ol, and their acceptable salts; and the 1-(1-azabicyclo[3.3.0]octan-5-yl)methan-1-ol, 2-(1-azabicyclo[3.3.0]octan-5-yl)ethan-1-ol or 2-(1-azabicyclo [3.3.0]octan-5-yl)propan-1-ol derivatives represented by Formula II may include (1R*)-1-(1-azabicyclo [3.3.0]octan-5-yl)-1-phenyl-1-[(3'S*)-3-tricyclo[2.2.1.0] heptyl]methan-1-ol, (1R*)-1-(1-azabicyclo[3.3.0]octan-5-yl)-1-phenyl-1-[(3'R*)-3-tricyclo[2.2.1.0]heptyl]methan-1-ol, 2-(1-azabicyclo[3.3.0]octan-5-yl)-1-(3-tricyclo[2.2.1.0]heptyl) ethan-1-ol, 2-(1-azabicyclo[3.3.0]octan-5-yl)-1-phenyl-1-(3-tricyclo[2.2.1.0]heptyl)propan-1-ol, 2-(1-azabicyclo [3.3.0]octan-5-yl)-2-methyl-1-phenyl-1-(3-tricyclo[2.2.1.0] heptyl)propan-1-ol, 1-(1-azabicyclo[3.3.0]octan-5-yl)-1-phenyl-1-(2-tricyclo[3.3.1.1$^{3.7}$]decyl)methan-1-ol, 1-(1-azabicyclo[3.3.0]octan-5-yl)-1-(2-naphthyl)-1-(2-tricyclo[3.3.1.1$^{3.7}$]decyl)methan-1-ol, 1-(1-azabicyclo[3.3.0]octan-5-yl)-1-(4-fluorophenyl)-1-(2-tricyclo[3.3.1.1$^{3.7}$]decyl)methan-1-ol, 1-(1-azabicyclo[3.3.0]octan-5-yl)-1-(4-dimethylaminophenyl)-1-(2-tricyclo[3.3.1.1$^{3.7}$]decyl) methan-1-ol, 1-(1-azabicyclo[3.3.0]octan-5-yl)-1-(4-methoxyphenyl)-1-(2-t ricyclo[3.3.1.1.$^{3.7}$]decyl)methan-1-ol, 1-(1-azabicyclo[3.3.0]-octan-5-yl)-1-(4-trifluoromethylphenyl)-1-(2-tricyclo[3.3.1.1$^{3.7}$]-decyl)methan-1-ol, 1-(1-azabicyclo[3.3.0]octan-5-yl)-1-(3-methoxyphenyl)-1-(2-tricyclo [3.3.1.1$^{3.7}$]decyl)methan-1-ol, 1-(1-azabicyclo [3.3.0]octan-5-yl)-1-(3-trifluoromethylphenyl)-1-(2-tricyclo

[3.3.1.1$^{3.7}$]decyl)methan-1-ol, 1-1(azabicyclo[3.3.0]-octan-5-yl)-1-(4-(2-methyl-1,3-thioxolane-2-yl)phenyl)-1-(2-tricyclo[3.3.1.1$^{3.7}$]decyl)methan-1-ol, 1-(4-acetylphenyl)-1-(1-azabicyclo[3.3.0]octan-5-yl)-1-(2-tricyclo[3.3.1.1$^{3.7}$]decyl)methan-1-ol, 1-(1-azabicyclo[3.3.0]octan-5-yl)-1-(2-methoxyphenyl)-1-(2-tricyclo[3.3.1.1$^{3.7}$]decyl)methan-1-ol, 1-(1-azabicyclo[3.3.0]-octan-5-yl)-1-(2-methylphenyl)-1-(2-tricyclo[3.3.1.1$^{3.7}$]decyl)methan-1-ol, 1-(1-azabicyclo [3.3.0]octan-5-yl)-1-(4-methylphenyl)-1-(2-tricyclo [3.3.1.1$^{3.7}$]decyl)methan-1-ol, 1-(1-azabicyclo[3.3.0]-octan-5-yl)-1-(4-biphenyl)-1-(2-tricyclo[3.3.1.1$^{3.7}$]decyl)methan-1-ol, and their acceptable salts.

The 3-piperidino-1-propanol derivatives, 1-(1-azabicyclo[3.3.0]octan-5-yl)methanol derivatives, 2-(1-azabicyclo[3.3.0]octan-5-yl)-1-ethanol derivatives and 2-(1-azabicyclo[3.3.0]octan-5-yl)-1-propanol derivatives which are structurally represented by Formulae I and II, and their acceptable salts are of excellent antiviral activity and also low toxicity, and so may be formulated as various pharmaceutical preparations.

When used for topical therapy, the compounds of the invention may be administered to tunica mucosa nasi, airways and the lungs in the form of aerosol and solutions for nebulizers, to the skin and mucosae in the form of a solution, ointment, cream, hydrogel or suppository (for both the rectum and vagina), and to an eye in the form eye lotion or ointment. These pharmaceuticals may be prepared in conventional manners and, if desired, may be used in combination with absorption enhancers such as bile salts, saponins, polyoxyethylene higher alcohol ethers, polyethylene glycol, dimethyl sulfoxide (DMSO) and laurocaplam. Although varying with the type of active compound, the conditions of human patients and the form of preparation, etc., these drugs may contain the active compound in amounts of 0.01 to 10%.

When administered to (human) patients, the compounds of the invention may be conventionally formulated into a solution, tablet, capsule, (fine) granules, buccal tablet or troche. If desired, they may be used in combination with absorption enhancers such as bile salts, saponins and polyoxyethylene higher alcohol ethers. Although varying depending on the type of active compound, the condition of the patient, the form of preparation, etc., they may generally be administered to the patient in doses within the range of 100 to 3,000 mg a day.

The compounds of the invention may also be compounded into injectable forms in conventional manner. They may then generally be administered to a human patient in doses of 30 to 1,000 mg a day, although depending on the type of compound, the conditions of the patient and the form of preparation.

The compounds of the invention may be easily synthesized by conventional methods. For example, 5-cyano-1-azabicyclo[3.3.0]-octane or 5-cyanomethyl-1-azabicyclo[3.3.0]octane which are synthesized by well-known methods, react with 2-tricyclo[3.3.1.1.$^{3.7}$]decyl lithium or 3-tricyclo[2.2.1.0]heptyl magnesium chloride, and substituted phenyl magnesium bromide to give a desired compound through a Grignard reaction.

## EXAMPLES

The present invention will now be illustrated with reference to preparative examples, pharmacological activity tests and some examples of pharmaceutical compositions. Firstly, Reference examples are provided of the preparation of starting materials. Percentage yields are by weight and ratios of solvents are by volume.

### Reference Example 1

### 3-chlorotricyclo[2.2.1.0]heptane

Bicyclo[2.2.1.0]hepta-2,5-diene (16.2 g, 0.176 mol) was cooled to -10°C, followed by blowing hydrogen chloride gas thereinto for 30 minutes. After this, the solution was stirred at 20°C for 40 hours in a hydrogen chloride gas atmosphere, and excess hydrogen chloride was distilled out of the reaction mixture under reduced pressure (20°C/100 mmHg and 1 hour) to obtain 22.7 g of a mixture of 5-chlorobicyclo[2.2.1]hept-2-ene with 3-chlorotricyclo[2.2.1.0]heptane in the form of a yellow oily product.

The obtained mixture (10.5 g, 0.0814 mol) was dissolved in methylene chloride (80 ml), followed by the gradual dropwise addition of titanium tetrachloride (0.89 ml, 8.1 mmol). Thereafter, the solution was stirred at 0-5°C for 2 hours, and the reaction mixture was poured in a mixed solution of ice water (100 ml) with methylene chloride (100 ml). After the separation of a methylene chloride layer, the product was washed with a saturated aqueous solution of sodium hydrogen carbonate (50 ml). The product was dried over anhydrous sodium sulfate, filtered, and distilled under normal pressure to remove a large part of methylene chloride. Finally, the residue was distilled under reduced pressure (at 103-105°C/120 mmHg) to obtain 8.97 g of the above-captioned compound in the form of a colorless oil product (in an 86.3% yield).

Boiling Point: 103-105°C (120 mmHg)

$^1$H-NMR spectrum ($CDCl_3$) δ ppm:

1.27-1.45 (6H, m, CH and $CH_2$)

1.99 (1H, d, J=10.7 Hz, $CH_2$)
2.06 (1H, s, CH)
3.91 (1H, s, Cl-CH)

Reference Example 2

3-piperidino-1-phenyl-1-propanone

Piperidine (20.2 g, 0.238 mol) was dissolved in toluene (200 ml), and sodium carbonate (6.31g, 59.5 mmol) was suspended in the solution. Then, 3-chloro-1-phenyl-1-propanone (20.0 g, 0.119 mol) was added to the suspension in small proportions, followed by a 2-hour stirring at 80°C. Following the cooling of the reaction mixture, the precipitated crystals were filtered, and the filtrate was concentrated to obtain 27.2 g of a crude product in the form of a brown oil product. Of this, 22.2 g was distilled under reduced pressure (95-98°C/0.6 mmHg) to obtain 16.1 g of the above-captioned compound in the form of a colorless oil product (in a 76.3% yield).
Boiling Point: 95-98°C (0.6 mmHg)
MS spectrum m/z:
EI/DI: 217 ($M^+$), 98 (base peak)
$^1$H-NMR spectrum ($CDCl_3$) $\delta$ ppm:
1.4-2.5 (10H, m, $CH_2$)
2.8-2.9 (2H, m, $COCH_2$)
3.2-3.3 (2H, m, $NCH_2$)
7.4-8.0 (5H, m, aromatic H)

Reference Example 3

5-cyano-1-azabicyclo[3.3.0]octane

1,7-dichloroheptan-4-one (20.0 g, 87.4 mmol) and 2-amino-2-methypropanenitrile (28.6 g, 326 mmol) were dissolved in methanol (96 g), followed by blowing ammonia gas into the solution at 20°C for 30 minutes. The solution was stirred at 20°C for 24 hours in an ammonia gas atmosphere. Methanol and ammonia were distilled off under reduced pressure, and methylene chloride (260 ml), water (133ml) and sodium hydroxide (25.7 g) were added to the residue. After the separation of an organic layer, an aqueous layer was extracted with methylene chloride (2 x 100 ml). The organic layers were combined together, dried over absolute sodium sulfate, and concentrated to obtain 33.80 g of a crude product, which was then distilled under reduced pressure (91°C/3 mmHg) to obtain 9.30 g of the above-captioned compound in the form of a colorless oil product (in a 78.1% yield).
Boiling Point: 91°C/3 mmHg
$^1$H-NMR spectrum ($CDCl_3$ $\delta$ ppm:
1.8-2.1 (8H, m, $CH_2$)
2.3-2.7 (4H, m, N-$CH_2$)

Reference Example 4

5-phenylcarbonyl-1-azabicyclo[3.3.0]octane

Phenyl lithium (a 1.8M cyclohexane-ether solution - 39.7 ml, 71.4 mmol) was added dropwise at -50 to -60°C over 15 minutes to a solution (35 ml) in ether of 5-cyano-1-azabicyclo [3.3.0]octane (8.10 g, 59.5 mmol) obtained in Reference Example 3. Following the completion of the dropwise addition, the solution was grdually heated to 20°C at which it was stirred for 1 hour. Water (40 ml) was added at 0-5°C to the reaction mixture in an ice bath, followed by the addition of ether (50 ml) and 10% sulfuric acid (80 ml), and the mixture was stirred at 0-5°C for a further 30 minutes. Sodium hydroxide (7.35 g) was added to the reaction mixture and stirred, followed by the separation of an organic layer. An aqueous layer was extracted with ether (150 ml x 2), and the organic layers were combined together. Ten (10) percent sulfuric acid (50 g) was added for a 1-hour stirring, and sodium hydroxide (5.0 g) was then added for ether removal. The aqueous layer was extracted with ether (100 ml x 2), dried over anhydrous magnesium sulfate, and concentrated to obtain 10.5 g of a crude product. This product was distilled under reduced pressure (110-113°C/0.4 mmHg) to obtain 9.00 g of the above-captioned product in the form of a light yellow oil product (in a 70.3% yield).

Boiling Point: 110-113°C (0.4 mmHg)
MS spectrum m/z:
EI/CI: 216 [$(M+1)^+$, base peak]
$^1$H-NMR spectrum ($CDCl_3$) δ ppm:
1.7-2.4 (8H, m, $CH_2$)
2.7-3.2 (4H, m, N-$CH_2$)
7.5-8.1 (5H, m, aromatic H)

Reference Example 5

5-cyanomethyl-1-azabicyclo[3.3.0]octane

In an argon atmosphere, pyrrolidinium perchlorate (25.0 g, 119 mmol), sodium hydroxide (4.77 g, 119 mmol) and ethanol (500 ml) were vigorously stirred at 3°C for 48 hours. The solvent was removed under reduced pressure to obtain 13.0 g of a colorless oil product. Cyanoacetic acid (16.0 g, 179 mmol) and dioxane (250 ml) were added to this product (without purification), followed by a 5-hour heating under reflux. The solvent was distilled off under reduced pressure, and ice water (100 ml) was added to the residue. After extraction with ether (300 ml), the product was washed with a saturated sodium chloride solution (100 ml), dried over anhydrous sodium sulfate, and distilled under reduced pressure for solvent removal, thereby obtaining 12.7 g of the above-captioned compound in the form of a colorless oil product (in a 71.2% yield).
Boiling Point: 86-87°C (1.2 mmHg)
MS spectrum m/z:
EI/DI: 150 ($M^+$), 110 (base peak)
CI/DI: 151 [$(M+1)^+$], 110 (base peak)
$^1$H-NMR spectrum ($CDCl_3$) 100δ ppm:
1.7-1.9 (8H, m, $CH_2$)
2.41 (2H, s, $CH_2CN$)
2.6-2.6 (2H, m, $NCH_2$)
3.1-3.2 (2H, m, $NCH_2$)
IR spectrum (neat) ν $cm^{-1}$:
2960 (C-H), 2250 (CN).

Reference Example 6

Methyl 2-(1-azabicyclo[3.3.0]octan-5-yl)ethane carboxylate

In an argon atmosphere, concentrated sulfuric acid (50 ml, 1.02 mmol) was added dropwise to a solution (150 ml) in ethanol of 5-cyanomethyl-1-azabicyclo[3.3.0]octane (12.5 g, 83.1 mmol) obtained in Reference Example 5. The reaction solution was heated under reflux for 16 hours. The reaction solution was poured into ice water (100 ml), neutralized with sodium hydrogen carbonate, and regulated to pH 11-12 with a 20% aqueous solution of sodium hydroxide. After extraction with chloroform (300 ml), the extract was dried over anhydrous sodium sulfate, distilled under reduced pressure for solvent removal, and distilled under reduced pressure to obtain 13.2 g of the above-captioned compound in the form of a colorless oil product (in an 86.5% yield).
Boiling Point: 82-84°C (1.6 mmHg)
MS spectrum m/z:
EI/DI: 183($M^+$), 110 (base peak)
$^1$H-NMR spectrum ($CDCl_3$) δ ppm:
1.6-2.0 (8H, m, $CH_2$)
2.45 (2H, s, $CH_2COOMe$)
2.5-2.6 (2H, m, N-$CH_2$)
3.0-3.1 (2H, m, N-$CH_2$)
3.66 (3H, s, $CH_3$)

Reference Example 7

2-(1-azabicyclo[3.3.0]octan-5-yl)methanal

In an argon atmosphere, a solution (150 ml) in ether of 2-(1-azabicyclo[3.3.0]octan-5-yl)ethane carboxylate

(5.20g, 28.4 mmol) was cooled down to -72°C or below, at which a DIBAL-H/toluene solution (56.8 ml, 85.1 mmol) was added dropwise to the solution, followed by a 15-minute stirring. At -50°C or below, 5N sulfuric acid (30 ml) was added to the reaction solution, which was then slowly heated to room temperature. The reaction solution was added to ice water (100 ml), neutralized with sodium hydrogen carbonate, and regulated to pH 11-12 with sodium hydroxide. After the insoluble matter was filtered out of the reaction solution, it was extracted with methylene chloride (500 ml), dried over anhydrous sodium sulfate, and distilled under reduced pressure for solvent removal. The residue was distilled under reduced pressure to obtain 2.94 g of the above-captioned compound in the form of a colorless oil product (in a 67.7% yield).
Boiling Point: 96°C (1.6 mmHg)
MS spectra m/z:
EI/DI: 153 ($M^+$), 110 (base peak)
CI/DI: 154 [$(M+1)^+$], 110 (base peak)
$^1$H-NMR spectrum ($CDCl_3$) $\delta$ ppm:
1.6-1.9 (8H, m, $CH_2$)
2.52 (2H, d, J=2.9 Hz, $CH_2CHO$)
2.6-2.7 (2H, m, N-$CH_2$)
3.0-3.1 (2H, m, N-$CH_2$)
9.80 (1H, t, J=2.69Hz, CHO)

Reference Example 8

2-(1-azabicyclo[3.3.0]octan-5-yl)-1-phenylpropan-1-one

In an argon atmosphere, a solution (50 ml) in ether of 5-cyanomethyl-1-azabicyclo[3.3.0]octane (28.0 g, 186 mmol) obtained in Reference Example 5 was cooled down to -50°C or below. At -50°C a phenyl lithium solution (284 ml, 216 mmol) was added dropwise to the resultant solution, followed by a 30-minute stirring. Methyl iodide (12.2 ml, 196 mmol) was added dropwise to the resulting solution, followed by a four-hour stirring. At -50°C a phenyl lithium solution (284 ml, 216 mmol) was added to the resulting solution, followed by a further 30-minute stirring. Methyl iodide (12.2 ml, 196 mmol) was added dropwise, followed by a further 2-hour stirring. At -50°C a phenyl lithium solution (388 ml, 295 mmol) was added dropwise, followed by a further 15-hour stirring. The reaction solution was added to ice water (500 ml), adjusted to pH 1 with concentrated sulfuric acid, and stirred for 3 hours. The solution was neutralized with sodium hydrogen carbonate, and then regulated to pH 11-12 with a 20% aqueous solution of sodium hydroxide. The reaction solution was extracted with methylene chloride, dried over anhydrous sodium sulfate, and distilled under reduced pressure for solvent removal. The residue was separated out and purified by column chromatography on silica gel (using ethyl acetate and n-hexane at 1:8 as the eluent to obtain 9.30 g of the above-captioned compound in the form of a colorless, viscous oil product (in a 19.5% yield).
MS spectrum m/z:
CI/DI: 245[$(m+1)^+$], 71, 89, 159, 175 (base peak)
$^1$H-NMR spectrum ($CDCl_3$) $\delta$ ppm:
1.24 (3H, s, $CH_3$)
1.4-2.3 (8H, m, $CH_2$)
2.6-2.7 (2H, m, N-$CH_2$)
2.9-3.0 (2H, m, N-$CH_2$)
7.3-9.1 (5H, m, aromatic H)

Preparative Example 1

(1R*)-1-phenyl-3-piperidino-1-((3'S*)-3-tricyclo[2.2.1.0] heptyl)propan-1-ol hydrochloride

Magnesium (4.54 g, 187 mmol) was washed with 1N hydrochloric acid (5 ml), then methanol (5 ml x 3) and then ether (5 ml x 3), and finally heated and dried under reduced pressure. The obtained active magnesium was suspended in ether (20 ml), and a small amount of 1,2-dibromoethane was added dropwise to the suspension with the application of heat under reflux, followed by the addition of a small amount of iodine pieces. After the confirmation of the fact that the color of iodine disappeared after the passing of 5 minutes, a solution (35 ml) in ether of 3-chlorotricyclo[2.2.1.0]heptane (8.00 g, 62.2 mmol) obtained in Reference Example 1 was added dropwise to the resulting solution with the application of heat under reflux over a period of 5 minutes. Following the completion of the dropwise addition, the resulting solution was heated under reflux for 30 minutes, and excess magnesium was removed by decantation. A solution (60 ml) in benzene of 3-piperidino-1-phenyl-1-propanone (7.50 g, 34.6 mmol) obtained in Reference Example 2 was added dropwise at 0°C for 10 minutes to the resulting solution. Following the completion of the dropwise addition, the solution was stirred at 60°C for 2 hours, and then poured into a saturated aqueous solution of ammonium chloride (80 ml) on an ice bath. After the separation of an ether layer, an aqueous layer was extracted with ether (200 ml x 3). The ether layers were combined together, dried over anhydrous magnesium sulfate, and concentrated to obtain 12.4 g of a crude product. This crude product was separated out and purified by column chromatography on silica gel (using benzene and THF at 10:1 as the eluent, thereby obtaining a main component. This component was converted into a salt with hydrogen chloride with 18% hydrogen chloride-ether, then recrystallized from n-propanol to obtain 2.40 g of the above-captioned compound in the form of a colorless, acicular crystal (in a 19.9% yield).

Sublimation Point: 227-230°C

MS spectrum m/z:

EI/DI: 311 ($M^+$), 98 (base peak)

$^1$N-NMR spectrum ($CDCl_3$) $\delta$ ppm:

0.89 (1H, d, J=10.3 Hz, CH)
1.0-1.9 (8H, m, $CH_2$ and CH)
1.3-3.6 (10H, m, $CH_2$)
2.2-3.2 (4H, m, $CH_2$)
3.05 (1H, brs, OH)
7.2-7.4 (5H, m, aromatic H)
11.5 (1H, brs, $N^+H$)

IR spectrum (KBr) $\nu$ $cm^{-1}$:

3350 (O-H), 2654, 2558 ($N^+H$)

Elementary Analysis:

| | |
|---|---|
| Calculated: | $C_{21}H_{30}ClNO$ (mol. wt.: 347.93) |
| | C 72.50, H 8.69, N 4.03 |
| Found: | C 72.28, H 8.85, N 4.11 |

Preparative Example 2

(1R*)-1-phenyl-3-piperidino-1-((3'R*)-3-tricyclo[2.2.1.0] heptyl)propan-1-ol hydrochloride

A by-product obtained by chromatography on silica gel in the process of preparing the compound of Preparative Example 1 was converted into a salt with hydrogen chloride according to the procedure of Preparative Example 1, thereby obtaining 0.230 g of the above-captioned compound in the form of a colorless, acicular crystal (in a 1.91% yield).

Sublimation Point: 185-190°C

MS spectrum m/z:

EI/DI: 311 ($M^+$), 98 (base peak)

$^1$N-NMR spectrum ($CDCl_3$) $\delta$ ppm:

0.7-2.2 (9H, m, $CH_2$ and CH)
1.7-3.6 (10H, m, $CH_2$)
2.4-3.1 (4H, m, $CH_2$)
2.99 (1H, brs, OH)
7.2-7.5 (5H, m, aromatic H)
11.6 (1H, brs, $N^+H$)

IR spectrum (KBr) $\nu$ $cm^{-1}$:

3322 (O-H), 2651, 2361 ($N^+H$)

Elementary Analysis:

| | |
|---|---|
| Calculated: | $C_{21}H_{30}ClNO$ (mol. wt.: 347.93) |
| | C 72,50, H 8.69, N 4.03 |
| Found: | C 72,30, H 8.76, N 4.02 |

Preparative Example 3

(1R*)-1-(1-azabicyclo[3.3.0]octan-5-yl)1-phenyl-1-[(3'S*)-3-tricyclo[2.2.1.0] heptyl]methan-1-ol hydrochloride

Magnesium (5.96 g, 0.245 mol) was washed with 1N hydrochloric acid (5 ml), then methanol (20 ml x 3) and then ether (50 ml x 3), and finally heated and dried under reduced pressure. The obtained active magnesium was suspended in ether (50 ml), and a small amount of 1,2-dibromoethane was added dropwise to the suspension with the application of heat under reflux, followed by the addition of a small amount of iodine pieces. After the confirmation of the fact that the color of iodine disappeared after the passing of 10 minutes, a solution (30 ml) in ether of 3-chlorotricyclo[2.2.1.0]heptane (10.5 g, 81.8 mmol) obtained in Reference Example 1 was added dropwise to the resulting solution with the application of heat under reflux over a period of 15 minutes. Following the completion of the dropwise addition, the resulting solution was heated under reflux for 30 minutes. The obtained solution was added dropwise at -5 to 0°C over a period of 30 minutes to a solution (60 ml) in ether of 5-phenylcarbonyl-1-azabicyclo[3.3.0]octane (8.00 g, 37.2 mmol) and, following the completion of the dropwise addition, the solution was stirred at 20°C for 2 hours. The reaction mixture was poured into a mixed solution of a saturated ammonium chloride (100 ml) with ether (100 ml) on an ice bath. After the separation of an ether layer, an aqueous layer was extracted with ether (150 ml x 3). The ether layers were combined together, dried over anhydrous magnesium sulfate, and concentrated to obtain 13.0 g of a crude product in the form of a yellow oil product. This crude product was separated out and purified by column chromatography on silica gel (using toluene and THF at 7:1→4:3 as the eluent, thereby obtaining 6.54 g of a free base of the above-captioned compound as a main component (in a 56.8% yield). This component was converted into a salt with hydrogen chloride with 18% hydrogen chloride-ether, then recrystallized from i-propanol to obtain 5.16 g of the above-captioned compound in the form of a colorless, prism crystal (in a 40.8% yield).

Melting Point: 203-206°C
MS spectrum m/z:
CI/DI: 310 $(M+1)^+$, 110 (base peak)
$^1$N-NMR spectrum ($CDCl_3$) δ ppm:
0.8-2.3 (16H, m, $CH_2$)
2.30 (1H, s, CH)
3.0-4.3 (4H, m, N-$CH_2$)
4.47 (1H, brs, OH)
7.2-7.3 and 7.8-8.0 (5H, m, aromatic H)
10.93 (1H, brs, $N^+H$)
IR spectrum (KBr) ν $cm^{-1}$:
3258, 3210 (O-H)
Elementary Analysis

| | |
|---|---|
| Calculated: | $C_{21}H_{28}ClNO$ (mol. wt.: 345.91) |
| | C 72.92, H 8.16, N 4.05 |
| Found: | C 72.62, H 8.35, N 3.92 |

Preparative Example 4

(1R*)-1-(1-azabicyclo[3.3.0]octan-5-yl)-1-phenyl-1-((3'R*)-3-tricyclo[2.2.1.0]heptyl)methanol hydrochloride

A by-product obtained by chromatography on silica gel in the process of preparing the compound of Preparative Example 3, i.e., 2.69 g of the free base of the above-captioned compound (in a 23.4% yield), was converted into a hydrochloride according to the procedure of Preparative Example 3 and recrystallized from i-propanol, thereby obtaining 2.36 g of the above-captioned compound in the form of a colorless prism crystal (in a 18.4% yield).

Melting Point: 192-195°C
MS spectrum m/z:
CI/DI: 310 $(M+1)^+$, 110 (base peak)
$^1$N-NMR spectrum ($CDCl_3$) δ ppm:
0.5-2.4 (15H, m, $CH_2$)
2.35 (1H, s, CH)
2.51 (1H, s, CH)
3.0-4.1 (4H, m, N-$CH_2$)

4.99 (1H, s, OH)
7.2-7.6 (5H, M, aromatic H)
11.61 (1H, brs, $N^+H$)

IR spectrum (KBr) ν $cm^{-1}$:
3320 (O-H), 2706, 2525 ($N^+H$)

Elementary Analysis:

| | |
|---|---|
| Calculated: | $C_{21}H_{28}ClNO$ (mol. wt.: 345.91) |
| | C 72.92, H 8.16, N 4.05 |
| Found: | C 72.75, H 8.41, N 3.84 |

Preparative Example 5

2-(1-azabicyclo[3.3.0]octan-5-yl)-1-(3-tricyclo[2.2.1.0] heptyl)ethan-1-ol

Magnesium (1.09 g, 45 mmol) was washed with 1N hydrochloric acid (2.0 ml), then methanol (5.0 ml x 3) and then ether (5.0 ml x 3), and dried under reduced pressure. In an argon atmosphere, ether (2.0 ml) and small amounts of 1,2-dibromoethane and iodine pieces were added to the obtained active magnesium. With the application of heat under reflux, the disappearance of the color of iodine was confirmed. Thereafter, a solution (3.0 ml) in ether of 3-chlorotricyclo [2.2.1.0]heptane (3.86 g, 30.0 mmol) obtained in Reference Example 1 was added dropwise to the solution, followed by a 30-minute stirring. While cooled down to 0°C or below, the resultant solution was added dropwise to a solution (10.0 ml) in ether of 2-(1-azabicyclo[3.3.0]octan-5-yl)methanol (0.919 g, 6.00 mmol) obtained in Reference Example 7 in an argon atmosphere, followed by a 5-hour stirring. The reaction solution was transferred into ice water (50 ml), and regulated to pH 11-12 with a 20% aqueous solution of sodium hydroxide. The resulting solution was extracted with methylene chloride (300 ml), dried over anhydrous sodium sulfate, and distilled under reduced pressure for solvent removal. The residue was separated out and purified by column chromatography on silica gel, using 10% methanol-chloroform as the eluent, thereby obtaining 1.03 g of the above-captioned compound in the form of a yellow oil product (in a 69.8% yield).

MS spectrum m/z:
EI/DI: 247 ($M^+$), 154, 111 (base peak)

$^1$H-NMR spectrum ($CDCl_3$) δ ppm:
0.7-1.4 (16H, m, $CH_2$)
2.12 (1H, brs, CH)
2.6-2.7 (2H, m, N-$CH_2$)
3.2-3.4 (2H, m, N-$CH_2$)
3.4-3.5 (1H, m, O-CH)

IR spectrum (neat) 115ν $cm^{-1}$:
3250 (O-H), 2940 (C-H)

Elementary Analysis:

| | |
|---|---|
| Calculated: | $C_{16}H_{25}NO$ (mol. wt.: 247.38) |
| | C 77.68, H 10.19, N 5.66 |
| Found: | C 77.34, H 10.32, N 5.38 |

Preparative Example 6

2-(1-azabicyclo[3.3.0]octan-5-yl)-1-phenyl-1-(3-tricyclo [2.2.1.0]heptyl)propan-1-ol

Magnesium (4.90 g, 202 mmol) was washed with 1N hydrochloric acid (5.0 ml), then methanol (5.0 ml x 3) and then ether (5.0 ml x 3), and dried under reduced pressure. In an argon atmosphere, ether (10.0 ml) and small amounts of 1,2-dibromoethane and iodine pieces were added to the obtained active magnesium. With the application of heat under reflux, the disappearance of the color of iodine was confirmed. Thereafter, a solution (20.0 ml) in ether of 3-chlorotricyclo [2.2.1.0]heptane (38.9 g, 303 mmol) obtained in Reference Example 1 was added dropwise to the solution, followed by a 30-minute stirring. While cooled down to 0°C or below, the resultant solution was added dropwise to a solution (50.0 ml) in ether of 2-(1-azabicyclo[3.3.0]octan-5-yl)-1-phenylpropan-1-one (9.00 g, 117 mmol) obtained in Reference Example 8 in an argon atmosphere, followed by a 3-hour stirring. The reaction solution was poured into ice water (100 ml), and regulated to pH 11-12 with a 20% aqueous solution of sodium hydroxide. The resulting solution was extracted with methylene chloride (300 ml), dried over anhydrous sodium sulfate, and distilled under reduced pressure for solvent removal. The

residue was separated out and purified by column chromatography on silica gel, using methylene chloride, methanol and THF at 8:1:1 as the eluent, thereby obtaining 1.21 g of the above-captioned compound in the form of a yellow, viscous oil product (in a 3.1% yield).

MS spectrum m/z:

CI/DI: 338 ($(M+1)^+$), 110, 256, 282 (base peak)

$^1$H-NMR spectrum ($CDCl_3$) δ ppm:

0.8-1.5 (7H, m, $CH_2$ and CH)
1.22 (3H, d, J=7.3 Hz, $CH_3$)
1.6-1.9 (8H, m, $CH_2$)
1.9-2.0 (1H, m, CH)
2.15 (1H, brs, CH)
2.24 (1H, t, J=7.3 Hz, O-CH)
2.5-2.7 (2H, m, N-$CH_2$)
2.8-3.3 (2H, m, N-$CH_2$)
7.1-7.5 (5H, m, aromatic H)

IR spectrum (KBr) ν $cm^{-1}$:

3100 (aromatic C-H), 2960 (C-H), 1470, 1450 (N-H)

Elementary Analysis:

| | |
|---|---|
| Calculated: | $C_{23}H_{31}NO$ (mol. wt.: 337.50) |
| | C 81.85, H 9.26, N 4.15 |
| Found: | C 81.63, H 9.03, N 4.00 |

Preparative Example 7

2-(1-azabicyclo[3.3.0]octan-5-yl)-2-methyl-1-phenyl-1-(3-tricyclo[2.2.1.0]heptyl)propan-1-ol

When preparing the compound referred to in Preparative Example 6, the above-captioned compound was obtained as a by-product in an amount of 0.617 g, that was in the form of a yellow, viscous oil product, (in a 1.58% yield).

MS spectrum m/z:

CI/DI: 352 ($(M+1)^+$), 110, 256, 282 (base peak)

$^1$H-NMR spectrum ($CDCl_3$) δ ppm:

0.8-1.5 (7H, m, $CH_2$ and CH)
1.25 (6H, s, $CH_3$)
1.6-1.9 (8H, m, $CH_2$)
1.9-2.2 (2H, m, CH)
2.24 (1H, t, J=7.3 Hz, O-CH)
2.5-2.7 (2H, m, N-$CH_2$)
2.8-3.3 (2H, m, N-$CH_2$)
7.1-7.5 (5H, m, aromatic H)

IR spectrum (KBr) ν $cm^{-1}$:

3100 (aromatic C-H), 2960 (C-H), 1470, 1450 (N-H)

Elementary Analysis:

| | |
|---|---|
| Calculated: | $C_{24}H_{33}NO$ (mol. wt.: 351.53) |
| | C 82.00, H 9.46, N 3.98 |
| Found: | C 81.85, H 9.31, N 4.06 |

Preparative Example 8

1-(1-azabicyclo[3.3.0]octan-5-yl)-1-phenyl-1-(2-tricyclo[3.3. 1.1$^{3.7}$]decyl)methan-1-ol

In an argon atmosphere, to a solution (200 ml) in ether of 5-cyano-1-azabicyclo[3.3.0]octane (5.45 g, 40.0 mmol) obtained in Reference Example 3, a 0.108 mM solution (600 ml, 64.8 mmol) in ether of 2-tricyclo[3.3.1.1$^{3.7}$]decyl lithium was added dropwise over a period of 2 hours at -50°C, followed by a 1-hour stirring at -50°C, and 18 hours at room temperature. The reaction solution was added to ice water (500 ml), adjusted to pH 3 with 3N aqueous hydrochloric acid, and stirred for 90 minutes. The solution was washed with ether (500 ml x 3), and adjusted to pH 9 with sodium hydrogen carbonate. After extraction with ether (500 ml x 3), the extract was dried over anhydrous sodium sulfate, distilled under reduced pressure for solvent removal to

obtain 9.02 g of 1-(1-aza-bicyclo[3.3.0]octan -5-yl)-1-(2-tricyclo[3.3. 1.1$^{3.7}$]decyl)-methan-1-one in the form of a yellow powder product (in an 82.5% yield).

In an argon atmosphere, to a solution (15 ml) in tetrahydrofuran of 1-(1-azabicyclo[3.3.0]octan-5-yl)-1-(2-tricyclo[3.3.1.1$^{3.7}$]decyl)methan-1-one (1.50 g, 5.49 mmol) a 1.1 M solutition (15 ml, 16.5 mmol) in tetrahydrofuran of phenyl magnesium bromide was added at -30°C to -40°C, followed by an 18-hour stirring at room temperature. The reaction solution was added to a saturated ammonium chloride solution, and adjusted to pH 10 with a 10% aqueous solution of sodium hydroxide. After extraction with ether (200 ml x 3), the extract was dried over anhydrous sodium sulfate, and distilled under reduced pressure for solvent removal. The residue was crystallized from methanol, and recrystallized from methylene chloride and methanol to obtain 1.42 g of the above-captioned compound in the form of a colorless prism crystal (in a 70.1% yield).

Melting Point: 105-108°C

MS spectrum m/z:

CI/dI: 352 ((M+1)$^+$), 110 (base peak)

Preparative Example 9

1-(1-azabicyclo[3.3.0]octan-5-yl)-1-(2-naphthyl)-1-(2-tricyclo [3.3.1.1$^{3.7}$]decyl)methan-1-ol

This compound was prepared by similar procedures as in Preparative Example 8 with the exception that 2-naphthyl magnesium bromide was used instead of phenyl magnesium bromine.

Melting Point: 166-169°C

MS spectrum m/z:

CI/DI: 402 ((M+1)$^+$), 110 (base peak)

Preparative Example 10

1-(1-azabicyclo[3.3.0]octan-5-yl)-1-(4-fluoropheyl)-1-(2-tricyclo [3.3.1.1$^{3.7}$]decyl)methan-1-ol

This compound was prepared by similar procedures as in Preparative Example 8 with the exception that 4-trifluorophenyl magnesium bromide was used instead of phenyl magnesium bromide.

Melting Point: 143-146°C

MS spectrum m/z:

CI/DI: 370 ((M+1)$^+$), 110 (base peak)

Preparative Example 11

1-(1-azabicyclo[3.3.0]octan-5-yl)-1-(4-fluoropheyl)-1-(2-tricyclo [3.3.1.1$^{3.7}$]decyl)methan-1-ol hydrochloride

The aqueous solution of 1-(1-azabicyclo[3.3.0]octan-5-yl)-1-(4-fluoropheyl)-1-(2-tricyclo [3.3.1.1$^{3.7}$]decyl)methan-1-ol (1.21 g, 3.27 mmol) obtained in Example 10 and 0.5 N aqueous hydrochloric acid (6.55 ml, 3.27 mmol) was filtered, and freeze-dried to obtain 1.42 g of the above-captioned compound in the form of a colorless powder.

MS spectrum m/z:

CI/DI: 370 ((M+1)$^+$), 110 (base peak)

Preparative Example 12

1-(1-azabicyclo[3.3.0]octan-5-yl)-1-(4-dimethylaminophenyl)-1-(2-tricyclo[3.3.1.1$^{3.7}$]decyl)methan-1-ol

This compound was prepared by similar procedures as in Preparative Example 8 with the exception that 4-dimethylaminophenyl lithium was used instead of phenyl magnesium bromide.

Melting Point: 160°C

MS spectrum m/z:

CI/DI: 395 ((M+1)$^+$), 110 (base peak)

Preparative Example 13

1-(1-azabicyclo[3.3.0]octan-5-yl)-1-(4-dimethylaminophenyl)-1-(2-tricyclo[3.3.1.$1^{3.7}$]decyl)methan-1-ol dihydrochloride

This compound was prepared by similar procedures as in Preparative Example 11 with the exception that 1-(1-azabicyclo[3.3.0]-octan-5-yl)-1-(4-dimethylaminophenyl)-1-(2-tricyclo[3.3.1.$1^{3.7}$]decyl)methan-1-ol was used instead of 1-(1-azabicyclo[3.3.0]octan-5-yl)-1-(4-fluorophenyl)-1-(2-tricyclo[3.3.1.$1^{3.7}$]-decyl)methan-1-ol. The above-captioned compound was then obtained in the form of a colorless powder.
MS spectrum m/z:
CI/DI: 395 ($(M+1)^+$), 110 (base peak)

Preparative Example 14

1-(1-azabicyclo[3.3.0]octan-5-yl)-1-(4-methoxyphenyl)-1-(2-tricyclo[3.3.1.$1^{3.7}$]decyl)methan-1-ol

This compound was prepared by similar procedures as in Preparative Example 8 with the exception that 4-methoxyphenyl magnesium bromide was used instead of phenyl magnesium bromide.
Melting Point: 158°C
MS spectrum m/z:
CI/DI: 382 ($(M+1)^+$), 110 (base peak)

Preparative Example 15

1-(1-azabicyclo[3.3.0]octan-5-yl)-1-(4-methoxyphenyl)-1-(2-tricyclo[3.3.1.$1^{3.7}$]decyl)methan-1-ol hydrochloride

This compound was prepared by similar Procedures as in Preparative Example 11 with the exception that 1-(1-azabicyclo[3.3.0]-octan-5-yl)-1-(4-methoxyphenyl)-1-(2-tricyclo[3.3.1.$1^{3.7}$]decyl)methan-1-ol was used instead of 1-(1-azabicyclo[3.3.0]octan-5-yl)-1-(4-fluorophenyl)-1-(2-tricyclo[3.3.1.$1^{3.7}$]-decyl)methan-1-ol. The above-captioned compound was then obtained in the form of a colorless powder.
MS spectrum m/z:
CI/DI: 382 ($(M+1)^+$), 110 (base peak)

Preparative Example 16

1-(1-azabicyclo[3.3.0]octan-5-yl)-1-(4-trifluoromethylphenyl)-1-(2-tricyclo[3.3.1.$1^{3.7}$]decyl)methan-1-ol

This compound was prepared by similar procedures as in Preparative Example 8 with the exception that 4-trifluorophenyl magnesium bromide was used instead of phenyl magnesium bromide.
Melting Point: 167-169°C
MS spectrum m/z:
CI/DI: 420 ($(M+1)^+$), 110 (base peak)

Preparative Example 17

1-(1-azabicyclo[3.3.0]octan-5-yl)-1-(4-trifluoromethylphenyl)-1-(2-tricyclo[3.3.1.$1^{3.7}$]decyl)methan-1-ol hydrochloride

This compound was prepared by similar procedures as in Preparative Example 11 with the exception that 1-(1-azabicyclo[3.3.0]-octan-5-yl)-1-(4-trifluoromethylphenyl)-1-(2-tricyclo[3.3.1.$1^{3.7}$]decyl)methan-1-ol was used instead of 1-(1-azabicyclo[3.3.0]octan-5-yl)-1-(4-fluorophenyl)-1-(2-tricyclo[3.3.1.$1^{3.7}$]-decyl)methan-1-ol. The above-captioned compound was then obtained in the form of a colorless powder.
MS spectrum m/z:
CI/DI: 420 ($(M+1)^+$), 110 (base peak)

Preparative Example 18

1-(1-azabicyclo[3.3.0]octan-5-yl)-1-(3-methoxyphenyl)-1-(2-tricyclo[3.3.1.1$^{3.7}$]decyl)methan-1-ol

This compound was prepared by similar procedures as in Preparative Example 8 with the exception that 3-methoxylphenyl magnesium bromide was used instead of phenyl magnesium bormide.
Melting Point: 152°C
MS spectrum m/z:
CI/DI: 382 ((M+1)$^+$), 110 (base peak)

Preparative Example 19

1-(1-azabicyclo[3.3.0]octan-5-yl)-1-(3-methoxyphenyl)-1-(2-tricyclo[3.3.1.1$^{3.7}$]decyl)methan-1-ol hydrochloride

This compound was prepared by similar procedures as in Preparative Example 11 with the exception that 1-(1-azabicyclo[3.3.0]-octan-5-yl)-1-(3-methoxyphenyl)-1-(2-tricyclo[3.3.1.1$^{3.7}$]decyl)methan-1-ol was used instead of 1-(1-azabicyclo[3.3.0]octan-5-yl)-1-(4-fluorophenyl)-1-(2-tricyclo[3.3.1.1$^{3.7}$]-decyl)methan-1-ol. The above-captioned compound was then obtained in the form of a colorless powder.
MS spectrum m/z:
CI/DI: 382 ((M+1)$^+$), 110 (base peak)

Preparative Example 20

1-(1-azabicyclo[3.3.0]octan-5-yl)-1-(3-trifluoromethylphenyl)-1-(2-tricyclo[3.3.1.1$^{3.7}$]decyl)methan-1-ol

This compound was prepared by similar procedures as in Preparative Example 8 with the exception that 3-trifluoromethylphenyl magnesium bromide was used instead of phenyl magnesium bromide.
Melting Point: 135-137°C
MS spectrum m/z:
CI/DI: 420 ((M+1)$^+$), 110 (base peak)

Preparative Example 21

1-(1-azabicyclo[3.3.0]octan-5-yl)-1-(3-trifluoromethylphenyl)-1-(2-tricyclo[3.3.1.1$^{3.7}$]decyl)methan-1-ol hydrochloride

This compound was prepared by similar procedures as in Preparative Example 11 with the exception that 1-(1-azabicyclo[3.3.0]-octan-5-yl)-1-(3-trifluoromethylphenyl)-1-(2-tricyclo[3.3.1.1$^{3.7}$]decyl)methan-1-ol was used instead of 1-(1-azabicyclo[3.3.0]octan-5-yl)-1-(4-fluorophenyl)-1-(2-tricyclo[3.3.1.1$^{3.7}$]-decyl)methan-1-ol. The above-captioned compound was then obtained in the form of a colorless powder.
MS spectrum m/z:
CI/DI: 420 ((M+1)$^+$), 110 (base peak)

Preparative Example 22

1-(1-azabicyclo[3.3.0]octan-5-yl)-1-(2-methoxyphenyl)-1-(2-tricyclo[3.3.1.1$^{3.7}$]decyl)methan-1-ol

This compound was prepared by similar procedures as in Preparative Example 8 with the exception that 2-methoxyhenyl magnesium bromide was used instead of phenyl magnesium bromide.
Melting Point: 133-136°C
MS spectrum m/z:
CI/DI: 382 ((M+1)$^+$), 110 (base peak)

Preparative Example 23

1-(1-azabicyclo[3.3.0]octan-5-yl)-1-(2-methoxyphenyl)-1-(2-tricyclo[3.3.1.$1^{3.7}$]decyl)methan-1-ol hydrochloride

This compound was prepared by similar procedures as in Preparative Example 11 with the exception that 1-(1-azabicyclo[3.3.0]-octan-5-yl)-1-(2-methoxyphenyl)-1-(2-tricyclo[3.3.1.$1^{3.7}$]decyl)methan-1-ol was used instead of 1-(1-azabicyclo[3.3.0]octan-5-yl)-1-(4-fluorophenyl)-1-(2-tricyclo[3.3.1.$1^{3.7}$]-decyl)methan-1-ol. The above-captioned compound was then obtained in the form of a colorless powder.
MS spectrum m/z:
CI/DI: 382 ($(M+1)^+$), 110 (base peak)

Preparative Example 24

1-(1-azabicyclo[3.3.0]octan-5-yl)-1-(2-methylphenyl)-1-(2-tricyclo[3.3.1.$1^{3.7}$]decyl)methan-1-ol

This compound was prepared by similar procedures as in Preparative Example 8 with the exception that 2-methylphenyl magnesium bromide was used instead of phenyl magnesium bromide.
Melting Point: 122°C
MS spectrum m/z:
CI/DI: 366 ($(M+1)^+$), 110 (base peak)

Preparative Example 25

1-(1-azabicyclo[3.3.0]octan-5-yl)-1-(2-methylphenyl)-1-(2-tricyclo[3.3.1.$1^{3.7}$]decyl)methan-1-ol hydrochloride

This compound was prepared by similar procedures as in Preparative Example 11 with the exception that 1-(1-azabicyclo[3.3.0]-octan-5-yl)-1-(2-methylphenyl)-1-(2-tricyclo[3.3.1.$1^{3.7}$]decyl)methan-1-ol was used instead of 1-(1-azabicyclo[3.3.0]octan-5-yl)-1-(4-fluorophenyl)-1-(2-tricyclo[3.3.1.$1^{3.7}$]-decyl)methan-1-ol. The above-captioned compound was then obtained in the form of a colorless powder.
MS spectrum m/z:
CI/DI: 366 ($(M+1)^+$), 110 (base peak)

Preparative Example 26

1-(1-azabicyclo[3.3.0]octan-5-yl)-1-(4-methylphenyl)-1-(2-tricyclo[3.3.1.$1^{3.7}$]decyl)methan-1-ol

This compound was prepared by similar procedures as in Preparative Example 8 with the exception that 4-methylphenyl magnesium bromide was used instead of phenyl magnesium bromide.
Melting Point: 142°C
MS spectrum m/z:
CI/DI: 366 ($(M+1)^+$), 110 (base peak)

Preparative Example 27

1-(1-azabicyclo[3.3.0]octan-5-yl)-1-(4-methylphenyl)-1-(2-tricyclo-[3.3.1.$1^{3.7}$]decyl)methan-1-ol hydrochloride

This compound was prepared by similar procedures as in Preparative Example 11 with the exception that 1-(1-azabicyclo[3.3.0]-octan-5-yl)-1-(4-methylphenyl)-1-(2-tricyclo[3.3.1.$1^{3.7}$]decyl)methan-1-ol was used instead of 1-(1-azabicyclo[3.3.0]octan-5-yl)-1-(4-fluorophenyl)-1-(2-tricyclo[3.3.1.$1^{3.7}$]-decyl)methan-1-ol. The above-captioned compound was then obtained in the form of a colorless powder. MS spectrum m/z:
CI/DI: 366 ($(M+1)^+$), 110 (base peak)

Preparative Example 28

1-(1-azabicyclo[3.3.0]octan-5-yl)-1-(4-biphenyl)-1-(2-tricyclo-[3.3.1.1$^{3.7}$]decyl)methan-1-ol

This compound was prepared by similar procedures as in Preparative Example 8 with the exception that 4-biphenyl magnesium bromide was used instead of phenyl magnesium bromide.
Melting Point: 148°C
MS spectrum m/z:
CI/DI= 428 ((M+1)$^+$), 110 (base peak)

Preparative Example 29

1-(1-azabicyclo[3.3.0]octan-5-yl)-1-(4-biphenyl)-1-(2-tricyclo[3.3.1.1$^{3.7}$]decyl)methan-1-ol hydrochloride

This compound was prepared by similar procedures as in Preparative Example 11 with the exception that 1-(1-azabicyclo[3.3.0]-octan-5-yl)-1-(4-biphenyl)-1-(2-tricyclo[3.3.1.1$^{3.7}$]decyl)methan-1-ol was used instead of 1-(1-azabicyclo[3.3.0]octan-5-yl)-1-(4-fluorophenyl)-1-(2-tricyclo[3.3.1.1$^{3.7}$]-decyl)methan-1-ol. The above-captioned compound was then obtained in the form of a colorless powder.
MS spectrum m/z:
CI/DI: 428 ((M+1)$^+$), 110 (base peak)

Preparative Example 30

1-(1-azabicyclo[3.3.0]octan-5-yl)-1-(4-(2-methyl-1,3-thioxolane-2-yl)phenyl)-1-(2-tricyclo[3.3.1.1$^{3.7}$]decyl)methan-1-ol

This compound was prepared by similar procedures as in Preparative Example 8 with the exception that 4-(2-methyl-1,3-thioxolane-2-yl)phenyl lithium was used instead of phenyl magnesium bromide.
Melting Point: 141°C
MS spectrum m/z:
CI/DI: 454 ((M+1)$^+$), 110 (base peak)

Preparative Example 31

1-(4-acetylphenyl)-1-(1-azabicyclo[3.3.0]octan-5-yl)-1-(2-tricyclo[3.3.1.1$^{3.7}$]decyl)methan-1-ol

In an argon atmosphere, to a solution (50 ml) in tetrahydrofuran of 1-(1-azabicyclo[3.3.0]octan-5-yl)-1-(4-(2-methyl-1,3-thioxolane-2-yl)phenyl)-1-(2-tricyclo[3.3. 1.1$^{3.7}$]decyl)methan-1-ol (1.00 g, 2.20 mmol) obtained in Preparative Example 30, a saturated aqueous solution of mercury (II) chloride (0.598 g, 2.20 mmol) was added, followed by a 5-minute stirring. To the reaction solution, 0.1 N aqueous sodium hydroxide (22.0 ml, 2.20 mmol) was added, followed by a 5-minute stirring, and the solution was extracted with methylene chloride. The extract was dried over anhydrous sodium sulfate, distilled under reduced pressure for solvent removal. The residue was separated out and purified by column chromatography on silica gel (using ether as the eluent), and recrystallized from methylene chloride and methanol to obtain 0.438 g of the above-captioned compound in the form of a colorless prism crystal (in a 50.5% yield).
Melting Point: 159°C
MS spectrum m/z:
CI/DI= 394 ((M+1)$^+$), 110 (base peak)

Preparative Example 32

1-(4-acetylphenyl)-1-(1-azabicyclo[3.3.0]octan-5-yl)-1-(2-tricyclo[3.3.1.1$^{3.7}$]decyl)methan-1-ol hydrochloride

This compound was prepared by similar procedures as in Preparative Example 11 with the exception that 1-(1-azabicyclo[3.3.0]-octan-5-yl)-1-(4-acetylphenyl)-1-(2-tricyclo[3.3.1.1$^{3.7}$]decyl)methan-1-ol was used instead of 1-(1-azabicyclo[3.3.0]octan-5-yl)-1-(4-fluorophenyl)-1-(2-tricyclo[3.3.1.1$^{3.7}$]-decyl)methan-1-ol. The above-captioned compound was then obtained in the form of a colorless powder.
MS spectrum m/z:

CI/DI: 394 ($(M+1)^+$), 110 (base peak)

Pharmacological Efficacy Test Examples

1. In vitro antiviral activities against influenza virus and cytotoxicity

Confluent MDCK cells (derived from the canine kidney) in 96-welled plates were infected with influenza virus (A/PR/8 strain) in the presence of various concentrations of the test compounds. After incubation, the virus-induced cytopathic effect (CPE), the inhibitory effect of CPE by the test compounds and cytotoxicity were microscopically observed. The virus titer ($TCID_{50}$) was determined from the CPE of the virus-infected culture. The antiviral effects of the test compounds were calculated from $TCID_{50}$ values of the test compound treated and control cultures and represented by $\Delta TCID_{50}$ ($log_{10}$). The test compounds were dissolved in MEM media, ethanol or DMSO, and diluted with MEM media supplemented with a 1.0% fetal bovine serum.

Table 1 - In-vitro antiviral activities of the test compounds againts influenza virus (A/PR/8 strain)

| Test Compounds | | Antiviral Activitiy ($\Delta TCID_{50}$)($log_{10}$) Comp. Concentrations, μg/ml | | |
|---|---|---|---|---|
| | 5 | 10 | 20 | 50 |
| Prep. Ex. 1 | | 0.44 (±) | >3.17 (+) | |
| Prep. Ex. 2 | | 0.50 (-) | 0.50 (±) | >3.00 (+) |
| Prep. Ex. 3 | | 0.83 (-) | 2.16 (-) | >3.33 (-) |
| Prep. Ex. 4 | | | 0.83 (±) | >3.00 (±) |
| Prep. Ex. 6 | | 0.00 (+) | 1.33 (+) | - |
| Prep. Ex. 8 | | 0.67 (+) | >3.50 (+) | |
| Prep. Ex. 9 | | - | - | |
| Prep. Ex. 10 | 2.00 (+) | >3.17 (+) | | |
| Prep. Ex. 11 | 1.00 (+) | >3.17 (+) | | |
| Prep. Ex. 13 | | 0.17 (+) | | |
| Prep. Ex. 15 | 0.00 (±) | >2.17 (+) | >3.50 (+) | |
| Prep. Ex. 17 | 0.33 (-) | 0.33 (+) | 0.33 (+) | |
| Prep. Ex. 19 | 0.00 (+) | 0.17 (+) | >3.17 (+) | |
| Prep. Ex. 21 | 0.00 (+) | 0.27 (+) | | |
| Prep. Ex. 23 | - | - | - | |
| Prep. Ex. 25 | | - | | |
| Prep. Ex. 27 | 0.17 (+) | 0.17 (+) | 2.17 (+) | |
| Prep. Ex. 29 | >3.00 (+) | >3.00 (+) | | |
| Prep. Ex. 30 | >3.00 (+) | >3.00 (+) | | |
| Prep. Ex. 32 | 0.50 (+) | 1.00 (+) | 1.16 | . |

In Table 1, the parentheses indicate the cytotoxicity of the test compounds, (-), (±) and (+) indicate no cytotoxicity, slight cytotoxicity and cytotoxicity with some observable antiviral effect, respectively. .

2. In vitro antiviral activities against herpes simplex virus type 1 and cytotoxicity

Confluent GMK cells (derived from the green monkey kidney) in 96-welled plates were infected with HSV-1 (Miyama strain) in the presence of various concentrations of the test compounds. After incubation, the virus-induced cytopathic effect (CPE), the inhibitory effect of CPE by the test compounds and cytotoxicity were microscopically observed. The virus titer ($TCID_{50}$) was determined from the CPE of the virus infected culture. The antiviral effects of the test compounds were calculated from $TCID_{50}$ values of the test compound treated and control cultures and represented by $\Delta TCID_{50}$ ($log_{10}$). The test compounds were dissolved in MEM media, ethanol or DMSO, and diluted with MEM media supplemented with a 1.0% fetal bovine serum.

Table 2 - In-vitro antiviral activities of the test compounds against herpes simplex virus type 1(Miyama strain)

| Test Compounds | | Antiviral Activity ($\Delta TCID_{50}$)($log_{10}$) Comp. Concentrations, μg/ml | | |
|---|---|---|---|---|
| | 5 | 10 | 20 | 50 |
| Prep. Ex. 1 | | 0.83 (-) | | >3.00 (+) |
| Prep. Ex. 2 | | 0.17 (-) | | >3.00 (+) |
| Prep. Ex. 3 | | 0.00 (-) | | >3.00 (-) |
| Prep. Ex. 4 | | 0.66 (-) | | 1.33 (±) |
| Prep. Ex. 6 | | 1.00 (±) | | |
| Prep. Ex. 8 | 0.50 (-) | 0.66 (-) | 2.66 (-) | |
| Prep. Ex. 9 | 1.17 | 2.17 (-) | - | |
| Prep. Ex. 10 | 0.83 (-) | 1.17 (-) | | |
| Prep. Ex. 11 | 0.00 (-) | 1.17 (-) | | |
| Prep. Ex. 13 | | 0.77 (-) | 3.00 (-) | |
| Prep. Ex. 15 | | 0.67 (-) | | |
| Prep. Ex. 17 | | 0.33 (-) | 0.33 (-) | |
| Prep. Ex. 19 | | 0.00 (-) | >2.00 (-) | |
| Prep. Ex. 21 | 0.50 (-) | 0.33 (-) | | |
| Prep. Ex. 23 | | 0.17 (+) | | |
| Prep. Ex. 25 | | >3.00 (+) | | |
| Prep. Ex. 27 | | 2.17 (-) | >3.00 (-) | |
| Prep. Ex. 29 | | >3.17 (+) | - | |
| Prep. Ex. 30 | | >3.17 (+) | | |
| Prep. Ex. 32 | | 0.33 (+) | | . |

In Table 2, the parentheses indicate the cytotoxicity of the test compounds, (-), (±) and (+) indicate no cytotoxicity, slight cytotoxicity and cytotoxicity with some observable antiviral effect, respectively.

Pharmaceutical Formulation Examples

Solution Composition 1

A solution was conventionally prepared according to the following recipe, and then packed in a glass ampoule.

| | |
|---|---|
| Compound of Preparative Example 3 | 1g |
| Physiological Saline | Suitable amount |
| | Total: 100 ml |

Ointment Composition 2

Ointment was conventionally prepared according to the following recipe, and then packed in an aluminum tube.

| | |
|---|---|
| Compound of Preparative Example 3 | 3g |
| White petroleum jelly | Suitable amount |
| | Total: 100 g |

Capsule Composition 3

A capsule composition was conventionally formulated according to the following recipe.

| | |
|---|---|
| Compound of Preparative Example 3 | 100 mg |
| Magnesium Stearate | 5 mg |
| Lactose | Suitable amount |
| | Total: 150 mg |

Tablet Composition 4

| | |
|---|---|
| Compound of Preparative Example | 100 mg |
| Sodium Lauryl Sulfate | 10 mg |
| Magnesium Stearate | 5 mg |
| Polyvinyl Pyrrolidone K30 | 11 mg |
| Carboxymethylcellulose (Ca) | 7 mg |
| Lactose | 60 mg |
| Corn Starch | Suitable amount |
| | Total: 210 mg |

The 3-piperidino-1-propanol derivatives, 1-(1-azabicyclo[3.3.0]octan-5-yl)methanol derivatives, 2-(1-azabicyclo[3.3.0]octan-5-yl)-1-ethanol derivatives or 2-(1-azabicyclo{3.3.0]octan-5-yl)-1-propanol derivatives according to the invention and their salts have excellent antiviral activites, and so are efficacious for preventing and treating various DNA virus, RNA virus and retrovirus infections. The compounds according to the invention, because they differ from existing antiviral substances in chemical structures, are again efficacious against infections caused by viruses that are resistant to existing antiviral substances.

## Claims

1. An antivirally active compound represented by the following formula (I):

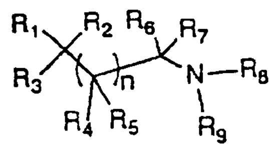

... (I)

wherein:

$R_1$ is a member selected from hydroxyl, amino and thiol,

$R_2$ is a member selected from hydrogen, α-naphthyl, β-naphthyl, and phenyl that has a substituent and is represented by the following formula:

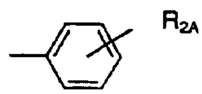

where $R_{2A}$ is a member selected from hydrogen, lower alkyl, halogeno, halogenoalkyl, $-OR_{2B}$, $-CO-R_{2C}$ and $-N-R_{2D}R_{2E}$, wherein $R_{2B}$ represents hydrogen or lower alkyl, and $R_{2C}$, $R_{2D}$ and $R_{2E}$ each represents lower alkyl,

$R_3$ is a member selected from monocycloalkyl, dicycloalkyl and tricycloalkyl that have each 6 to 16 carbon atoms and may have a substituent,

$R_{4-7}$ are each a member selected from hydrogen and lower alkyl,

$R_{8-9}$ are each lower alkyl, or may form a six-membered ring, or may form a five-membered ring with $R_6$ and $R_7$, and

n is 0 or an integer.

2. An antivirally active compound represented by the following general formula II:

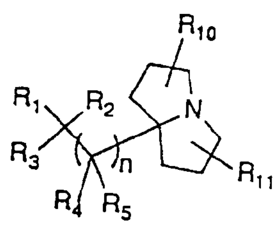

... (II)

wherein:

$R_1$ is a member selected from hydroxyl, amino and thiol,

$R_2$ is a member selected from hydrogen, α-naphthyl, β-naphthyl, and phenyl that has a substituent and is represented by the following formula:

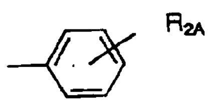

where $R_{2A}$ is a member selected from hydrogen, lower alkyl, halogeno, halogenoalkyl, $-OR_{2B}$, $-CO-R_{2C}$ and $-N-R_{2D}R_{2E}$, wherein $R_{2B}$ represents hydrogen or a lower alkyl, and $R_{2C}$, $R_{2D}$ and $R_{2E}$ each represents lower alkyl,

$R_3$ is a member selected from monocycloalkyl, dicycloalkyl and tricycloalkyl that have each 6 to 16 carbon atoms and may have a substituent,

$R_{4-7}$ each each a member selected from hydrogen and lower alkyl,

$R_{8-9}$ are each lower alkyl, or may form a six-membered ring, or may form a five-membered ring with $R_6$ and $R_7$,

$R_{10-11}$ are each lower alkyl, and

n is 0 or a natural number.

3. A compound as claimed in Claim 1, which is selected from (1R*)-1-phenyl-3-piperidino-1-((3'S*)-3-tricyclo [2.2.1.0]heptyl)-1-propanol and (1R*)-1-phenyl-3-piperidino-1-((3'R*)-3-tricyclo[2.2.1.0]heptyl)-1-propanol.

4. A compound as claimed in Claim 2, which is selected from (1R*)-1-(1-azabicyclo [3.3.0]octan-5-yl)-1-phenyl-1-[(3'S*)-3-tricyclo[2.2.1.0] heptyl]methan-1-ol, (1R*)-1-(1-azabicyclo[3.3.0]octan-5-yl)-1-phenyl-1-[(3'R*)-3-tricyclo[2.2.1.0]hepthyl]methan-1-ol, 2-(1-azabicyclo[3.3.0]octan-5-yl)-1-(3-tricyclo[2.2.1.0]heptyl) ethan-1-ol, 2-(1-azabicyclo[3.3.0]octan-5-yl)-1-phenyl-1-(3-tricyclo [2.2.1.0]heptyl)propan-1-ol, 2-(1-azabicyclo [3.3.0]octan-5-yl)-2-methyl-1-phenyl-1-(3-tricyclo[2.2.1.0] heptyl)propan-1-ol, 1-(1-azabicyclo[3.3.0]octan-5-yl)-1-phenyl-1-(2-tricyclo[3.3.1.1$^{3.7}$]decyl)methan-1-ol, 1-(1-azabicyclo[3.3.0]octan-5-yl)-1-(2-naphthyl)-1-(2-tricyclo [3.3.1.1$^{3.7}$]decyl)methan-1-ol, 1-(1-azabicyclo[3.3.0]octan-5-yl)-1-(4-fluorophenyl)-1-(2-tricyclo-[3.3.1.1$^{3.7}$]decyl)methan-1-ol, 1-(1-azabicyclo[3.3.0]octan-5-yl)-1-(4-dimethylaminophenyl)-1-(2-tricyclo[3.3.1.1$^{3.7}$]decyl) methan-1-ol, 1-(1-azabicyclo[3.3.0]octan-5-yl)-1-(4-methoxyphenyl)-1-(2-tricyclo[3.3.1.1.$^{3.7}$]decyl)methan-1-ol, 1-(1-azabicyclo[3.3.0] -octan-5-yl)-1-(4-trifluoromethylphenyl)-1-(2-tricyclo [3.3-1.1$^{3.7}$]-decyl)methan-1-ol, 1-(1-azabicyclo[3.3.0]octan-5-yl)-1-(3-methoxyphenyl)-1-(2-tricyclo[3.3.1.1$^{3.7}$]dcyl)methan-1-ol, 1-(1-azabicyclo [3.3.0]octan-5-yl)-1-(3-trifluoromethylphenyl)-1-(2-tricyclo [3.3.1.1$^{3.7}$]decyl)methan-1-ol, 1-1(azabicyclo[3.3.0]-octan-5-yl)-1-(4-(2-methyl-1,3-t hioxolane-2-yl)phenyl)-1-(2-tricyclo[3.3.1.1$^{3.7}$]decyl)methan-1-ol, 1-(4-acetylphenyl)-1-(1-azabicyclo[3.3.0]octan-5-yl)-1-(2-tricyclo[3.3.1.1$^{3.7}$] decyl)methan-1-ol, 1-(1-azabicyclo [3.3.0]octan-5-yl)-1-(2-methoxyphenyl)-1-(2-tricyclo [3.3.1.1$^{3.7}$]decyl)methan-1-ol, 1-(1-azabicyclo[3.3.0]-octan-5-yl)-1-(2-methylphenyl)-1-(2-tricyclo[3.3.1.1$^{3.7}$]decyl)methan-1-ol, 1-(1-azabicyclo [3.3.0]octan-5-yl)-1-(4-methylphenyl)-1-(2-tricyclo [3.3.1.1$^{3.7}$]decyl)methan-1-ol, 1-(1-azabicyclo [3.3.0]-octan-5-yl)-1-(4-biphenyl)-1-(2-tricyclo[3.3.1.1$^{3.7}$] decyl)methan-1-ol.

5. An antivirally active composition which comprises a suitable pharmaceutical carrier and as an active ingredient a therapeutically effective amount of a compound as defined in any one of Claims 1-4 or its pharmaceutically acceptable salt.

6. An antivirally active composition as claimed in Claim 5, which further contains a polyoxyethylene higher alcohol ether or a surface active agent.

7. An antivirally active composition as defined in Claim 5 or 6, which is usable for preventing and treating viral infections ascribable to various DNA viruses, RNA viruses and retroviruses.

8. An antivirally active composition as claimed in Claim 5 or 6, which is usable for treating viral infections ascribable to Orthomyxoviridae viruses inclusive of the influenza virus.

9. An antivirally active composition as claimed in Claim 5 or 6, which is usable for treating viral infections ascribable to Herpes virus viruses inclusive of the HIV-1 virus.

10. An antivirally active composition as claimed in Claim 5 or 6, which is usuable for treating viral infections ascribed to Retroviridae viruses inclusive of the AIDS virus.

European Patent Office

# PARTIAL EUROPEAN SEARCH REPORT

which under Rule 45 of the European Patent Convention shall be considered, for the purposes of subsequent proceedings, as the European search report

Application Number
EP 93 30 1415
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X,D | ACTA VIROL.<br>vol. 28, 1984,<br>pages 501 - 7 'Antiviral Activity of Norakin (Triperiden) and Related Anticholinergic Antiparkinsonism Drugs'<br>* summary *<br>* page 501, line 10 - line 15 *<br>* page 504, line 17 - line 18 *<br>--- | 1,3,5-10 | A61K31/445<br>A61K31/40 |
| X | US-A-3 553 225 (KAISER ET AL.)<br>* claim 1 *<br>* examples 1-3 *<br>--- | 1,3,5-10 | |
| A | PHARMAZIE<br>vol. 43, no. 7, 1988,<br>pages 489 - 92 'Triperiden (Norakin): Biotransformation in vivo und in vitro'<br>* page 490, left column, line 3 - line 7 *<br>* page 491, left column, line 40 - right column, line 11 *<br>---<br>-/-- | 1-10 | |

TECHNICAL FIELDS SEARCHED (Int. Cl. 5)

A61K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 13 MAY 1993 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number
EP 93 30 1415
Page 2

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 5) |
| A,D | PATENT ABSTRACTS OF JAPAN<br>vol. 11, no. 193 (C-430)20 June 1987<br>* abstract *<br>& JP-A-62 016 487 (SANWA KAGAKU KENKYUSHO)<br>----- | 1-10 | |
| | | | TECHNICAL FIELDS SEARCHED (Int. Cl. 5) |

EPO FORM 1503 03.82 (P04E10)

EP 93 30 1415 -C-

Claims searched incompletely : 1-10

Reason : For economic reasons (cf. Guidelines C-III 3.6-7, Art. 84 EPC), the search has been limited to the compounds of the specific preparative examples mentioned in the description, and pharmaceutical compositions thereof.